# EUROPÄISCHE PATENTSCHRIFT

(11) **EP 0 709 679 B1**
(45) Veröffentlichungstag und Bekanntmachung des Hinweises auf die Patenterteilung: **07.07.1999**
(21) Anmeldenummer: 95890187.8
(22) Anmeldetag: 18.10.1995
(51) Int. Cl.: G01N 33/543, G01N 33/50, C12Q 1/00

(54) **Verfahren und mizellares System zur Bestimmung einer Substanz**
Method and micelle system to determine a substance
Méthode et système micellulaire pour déterminer une substance

(30) Priorität: 24.10.1994 CH 318194
(43) Veröffentlichungstag der Anmeldung: 01.05.1996
(73) Patentinhaber: AVL Medical Instruments AG, 8207 Schaffhausen (CH)
(72) Erfinder: Spichiger-Keller, Ursula, Dr., CH-8804 Au-Wädenswil (CH); Vaillo, Eva, Dr., CH-8006 Zürich (CH)
(74) Vertreter: Krause, Walter, Dr. Dipl.-Ing.

(56) Entgegenhaltungen:
- EUROPEAN JOURNAL OF BIOCHEMISTRY, Bd. 155, Nr. 3, 1.März 1986 CAMBRIDGE UK, Seiten 453-468, K. MARTINEK ET AL. 'Micellar enzymology'
- ANALYTICAL BIOCHEMISTRY, Bd. 181, 1989 NEW YORK NY USA, Seiten 145-148, A.V. KABANOV ET AL. 'A new way in homogenous immunoassay: reversed micellar systems as a medium for analysis.'

## Beschreibung

Die vorliegende Erfindung betrifft ein Verfahren, die Anwendungen des Verfahrens und eine Schichtstruktur zur Bestimmung einer Substanz gemäss den Patentansprüchen 1, 10 - 14 und 15.

### 1. Einleitung

Chemische Sensoren zur Bestimmung von chemischen Substanzen, sog. Analyten, in Proben oder Specimen umfassen ein ganzes Spektrum von thermodynamisch reversiblen oder regenerierbaren Messsonden. Diese Messsonden bestehen obligatorisch aus mindestens einem Erkennungselement, mindestens einem Transduktionselement und, idealerweise, mind. einem Kalkulations- und Anzeigeelement. Das Erkennungselement ist meistens in einer Schichtstruktur implantiert; der Analyt wird selektiv von anderen Substanzen in der Probe / im Specimen isoliert und in die Schicht extrahiert. Erkennungsmoleküle werden auch Wirtsmoleküle genannt und erkennen den Analyten, auch Gastmolekül genannt, selektiv. Der Erkennungsprozess, die Wechselwirkung zwischen Wirt- und Gastmolekül, wird durch einen Transduktionsprozess in ein Signal überführt, das quantifiziert werden kann. Biosensoren vertreten eine Untergruppe der chemischen Sensoren, bei welchen das Erkennungselement aus einer biologisch aktiven Substanz besteht wie Enzyme, Peptide, Rezeptoren, Antikörper, Zellorganellen, usw.

Chemische Sensoren und Biosensoren verzeichnen ein wachsendes Entwicklungspotential und eine wachsende Bedeutung in der Analytik (Umwelt- und Lebensmittelanalytik, medizinische und biotechnologische Analytik). Die Merkmale dieser Sensoren sind:
- Kostengünstige und schnelle Analysen,
- kontinuierliche Messtechnik direkt im Specimen,
- Mobilität der Sonden und Messsysteme,
- Einsatz an schwer zugänglichen Orten und Stellen mit erhöhtem Risiko,
- selektiver Einsatz von sogenannten massgeschneiderten, spezifischen Systemen.

### 2. Stand der Technik

### 2.1. Erkennungsmoleküle

Als chemische Erkennungsmoleküle im engeren Sinne sind synthetische Substanzen bekannt, die spezifisch zu diesem Zwecke geplant sind (W. Simon, U.E. Spichiger, Development and Application of Ion-Selective Electrodes, International Laboratory, September 1991, 35-43). In Biosensoren werden im Gegensatz dazu Moleküle eingesetzt, die bereits aus biologischen Prozessen bekannt sind oder doch in biologischen Systemen produziert werden. Einige davon sind Enzyme, synthetische und genmanipulierte Enzyme; Antikörper, Antikörperfragmente, synthetische und genmanipulierte Antikörper; Rezeptoren und deren Hybride; Peptide, Kohlenhydrate, Lipide und deren Hybride.

### 2.2. Design von Sensoren

Erkennungsmoleküle werden bekannterweise auf zwei verschiedene Arten in der chemischen Sonde implementiert:
1) durch Immobilisation an der Oberfläche des Trägermaterials (elektrostatische, kovalente und adhesive Wechselwirkungen u.a.), welche sowohl mit apolaren wie auch polaren Phasen kontaktiert werden können (Hall, G.F., Turner, A.P.F., J. Cell. Biochem., Suppl. 14B (1990) 354; Hall, G.F. et al., Anal. Chim. Acta 213 (1988) 113-119).
2) durch Einbringen in polare oder apolare Bulk-Membranen, sodass die molekulare Erkennung in oder nahe der Grenzfläche und nicht auf der Oberfläche der Membran stattfindet. Beispiele dafür sind verschiedentlich beschrieben worden (Seiler, K., Simon, W., Anal. Chim. Acta, 266 (1992) 73-87; Patentschrift EP-A-0153641; Patentschrift JP-A-2082152).
Der Transduktionsprozess besteht in der Umsetzung des chemischen Erkennungsschrittes in eine physikalische Messgrösse. Im Falle der Optoden (optische Sensoren) ist die Messgrösse z.B. ein optisches Signal, im Falle der Elektroden (elektrochemische Sensoren) besteht sie aus einem elektrischen Signal.

### 2.3 Bekannte Sensoren

Chemische Sensoren und Biosensoren werden für die quantitative Analyse verschiedener Analyte eingesetzt, so z.B.
2.3.1 für Ionen und geladene Moleküle:
   - Ionen-selektive Elektroden
   - Potentiometrische Elektroden
   - Ionen-selektive Optoden
2.3.2 für ungeladene, geladene oder neutrale Moleküle:
   - Optoden
   - Amperometrische, enzymatische Elektroden
   - Biosensoren, Immunsensoren

### 2.4 Probleme und Nachteile bekannter Sensorsysteme

Für Sensoren, bei welchen das Erkennungsmolekül an der Oberfläche immobilisiert ist (vgl. 2.2.1) und gegen die Probe exponiert wird, ist die Umgebung des Erkennungsprozesses schlecht definiert. Die Qualität entspricht in der Regel nicht den Anforderungen, welche an einen zuverlässigen Sensoren gestellt werden. Derartige Mängel sind beispielsweise die Lebensdauer, die Invarianz gegen pH, Proteine, allgemeine unspezifische Einflüsse durch Specimen- und Probematrix bzw. Ionenstärke. Es treten unspezifische Wechselwirkungen mit dem Probe-/Specimenhintergrund auf. Die Regeneration der Erkennungsschicht hat den Verlust von kostspieligen Verbindungen, wie etwa Antigene und Antikörper, zur Folge.

Bei Sensoren, bei welchen das Erkennungsmolekül in einer Schichtstruktur implantiert ist (vgl. 2.2.2) sind diese Nachteile aufgehoben. Bei dieser Art von Biosensoren wird aber in der Regel die Transduktionsschicht von der Erkennungsschicht abgetrennt. Dadurch geht ein Teil des Signals durch Diffusion verloren; das Ansprechverhalten wird langsamer und die Signalausbeute geringer, wie dies im Fall der Harnstoff-Optode von Stamm beschrieben wird (C. Stamm et al., Anal. Chim. Acta, 282 (1993), 229-237).

Im weiteren ist bekannt, dass sich Biomoleküle in organischen Lösungsmitteln mittels Umkehrmizellen solubilisieren lassen und dabei ihre biologische Aktivität behalten (Luisi, P.L. et al., Methods in Enzymologiy, Vol. 136 (1987) 188-216, Acad. Press Inc.); Martinek, K. et al., Eur.J. Biochem. 155 (1986) 453-468). Die beschriebenen Drei-Komponenten-Systeme, bestehend aus einem Tensid, einem polaren, hydrophilen und einem apolaren, lipophilen Lösungsmittel und werden durch das Verhältnis von Wasser, als polarem Lösungsmittel, zu Tensid mit dem Parameter Wₒ charakterisiert. Die Funktionsfähigkeit der darin solubilisierten Enzyme wurde in Abhängigkeit des Wassergehaltes untersucht (Han, D. et al., Biocatalysis, Vol. 4 (1990), 153-161).

Aufgabe der vorliegenden Erfindung ist es, Verfahren und Schichtstrukturen zur Bestimmung von Substanzen anzugeben, bei welchen ein mizellares Erkennungssystem gebildet und in einer Schichtstruktur eingebaut wird. Beim Kontaktieren der zu bestimmenden Substanz mit der Schichtstruktur findet ein Erkennungsschritt statt, welchem ein Transduktionsschritt folgt, wodurch eine Messgrösse erzeugt wird. Aus dieser Messgrösse wird die gewünschte Information über die Substanz ermittelt.

Durch den Lösungsansatz, die Erkennungskomponente in einem mizellaren System einzubauen und daraus ein mizellares Erkennungssystem zu konstruieren, werden die aufgeführten Nachteile (vgl. 2.4) weitgehend aufgehoben. Der amphiphile Charakter dieser Systeme ermöglicht die Vereinigung der bisher bekannten Erkennungsschicht und Transduktionsschicht in einer einzigen Schicht.

Erfindungsgemäss wird diese Aufgabe mittels Verfahren gemäss dem Wortlaut der Patentansprüche 1 - 9 und mittels Schichtstrukturen gemäss dem Wortlaut der Patentansprüche 15 - 19 gelöst. Der Erfindung werden die nachstehenden Begriffsdefinitionen zu Grunde gelegt.

### 3. Begriffsdefinitionen

### 3.1 Substanz

Als Substanz werden alle neutralen (ungeladenen oder zwitterionischen) und geladenen Verbindungen, sowie Radikale und Isotope definiert, über welche Informationen gewonnen werden sollen.

### 3.2 Erkennungskomponente

Erkennungskomponenten sind Verbindungen, welche eine spezifische Wechselwirkung mit der zu bestimmenden Substanz, bzw. dem Gastmolekül, eingehen.

### 3.3 Mizellares System

Als mizellares System wird ein System definiert, welches sich aus drei Komponenten zusammensetzt, die in einem bestimmten quantitativen Verhältnis zueinander stehen. Die drei Komponenten sind: eine lipophile Phase, eine hydrophile Phase und mindestens eine grenzflächenaktive Substanz.

### 3.4 Mizellares Erkennungssystem

Als mizellares Erkennungssystem wird die Kombination aus mindestens einer Erkennungskomponente (3.2) und aus einem mizellaren System (3.3) definiert.

### 3.5 Erkennungsschritt 1. Art

Als Erkennungsschritt 1. Art wird ein Schritt definiert, bei dem eine selektive Wechselwirkung zwischen der Substanz und der Erkennungskomponente stattfindet.

### 3.6 Erkennungsschritt 2. Art

Als Erkennungsschritt 2. Art wird ein Schritt definiert, bei dem der Erkennungsschrittes 1. Art zu einer weiteren, bedingt selektiven Reaktion mit einer weiteren Erkennungskomponente führt.

### 3.7 Transduktionsschritt

Als Transduktionsschritt wird ein Schritt oder eine Folge von Schritten bezeichnet, bei welchem der Erkennungsschritt 1. Art der Substanz entweder direkt oder über einen oder mehrere Erkennungsschritte 2. Art zu einer quantifizierbaren Messgrösse führt, wovon jeder der Erkennungsschritte 2. Art Teil der Transduktion bildet.

### 3.8. Schichtstruktur

In der Schichtstruktur bildet die Kombination des Erkennungsschrittes 1. Art (3.5) und des Transduktionsschrittes (3.7) eine einzige Schicht, wovon der Transduktionsschritt mindestens teilweise in dieser einzigen Schicht stattfindet. Zusätzliche Hilfsschichten, wie z.B. für den Ionenausschluss, zur Verbesserung der Bioverträglichkeit, zur Förderung der Gaspermeabilität usw. sind zulässig. Die Kombination dieser Schichten wird als Schichtstruktur definiert.

### 3.9. Cocktail

Als Cocktail wird eine Mischung der Komponenten des mizellaren Erkennungssystems (3.4) mit mindestens einem Hilfsstoff definiert.

Die Erfindung wird im weiteren an Hand der Fig. 1 - 5 näher beschrieben. Es zeigen:
- Fig. 1: Flussdiagramm eines Verfahrens zur Bestimmung einer Substanz
- Fig. 2: Schematische Darstellung eines mizellaren Erkennungssystems mit Umkehrmizellen
- Fig. 3: Schematische Darstellung eines mizellaren Erkennungssystems mit normaler Mizellanordnung
- Fig. 4: Schematische Darstellung des eingebrachten mizellaren Erkennungssystems als Schicht einer Schichtstruktur gemäss Prozedur A
- Fig. 5: Schematische Darstellung eines Erkennungsschrittes 1. Art gekoppelt an einen Erkennungsschrittes 2. Art in einer Schicht

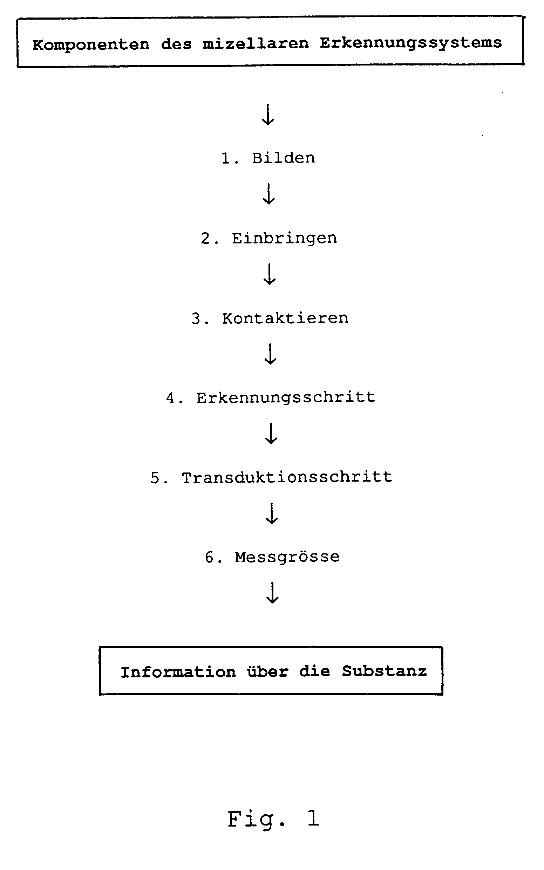

Das erfindungsgemässe Verfahren wird unter Bezug auf das Flussdiagramm (Fig. 1) näher erläutert, wobei in Fig. 1 die allgemeinen Schritte dargestellt sind. Dabei werden für die Beschreibung der verschiedenen Verfahrensschritte diejenigen Referenzzahlen des Flussdiagrammes verwendet, die für die Gliederung der einzelnen Schritte angeführt sind.

### 4. Flussdiagramm

Fig. 1 zeigt das Flussdiagramm für ein Verfahren zur Bestimmung einer Substanz, welches durch die Schritte 1 - 6 nachstehend beschrieben wird:

### Komponenten des mizellaren Erkennungssystems

Das mizellare Erkennungssystem besteht grundsätzlich aus einem mizellaren System und mindestens einer Erkennungskomponente. Liegt lediglich eine Erkennungskomponente vor, so führt dies zu einem Erkennungsschritt 1. Art wie später detailliert beschrieben wird. Liegen mehrere Erkennungskomponenten vor, so führen diese im allgemeinen zu Erkennungschritten der 1. Art sowie der 2. Art. Die Bestandteile eines mizellaren Erkennungssystem sind:

*Zwei nicht-mischbare Phasen,* wovon die eine einen lipophilen und die andere einen hydrophilen Charakter aufweist:
1) Die *lipophile Phase* weist eine geringe Polarität auf und ist beispielsweise ein organisches Lösungsmittel wie Tetrahydrofuran, Cyclohexan, Cyclohexanon, usw.; beliebige Mischungen von organischen Lösungsmitteln, usw.; ein Weichmacher, ein Polymer, Silikonöl, Methylenchlorid.
2) Die *hydrophile Phase* besteht aus Lösungsmitteln erhöhter Polarität wie etwa H₂O, wässrige Pufferlösungen, mit H₂O mischbare Lösungsmittel, wie etwa Acetonitril; oder Hydrogele.

Diese zwei nicht-mischbaren Phasen haben gemeinsam folgende beiden Funktionen zu erfüllen, was die Abstimmung der beiden Phasen aufeinander im wesentlichen bestimmt: Sie bestimmen die Extraktionseigenschaften und sind Lösungsmittel für die Komponenten. Sie bestimmen gemeinsam die Viskosität der zu erstellenden Schicht. Sie bestimmen die Lokalisierung der gelösten Komponenten, d.h. die Verteilung der Komponenten zwischen den beiden Phasen.
3) eine *grenzflächenaktive Substanz,* natürlicher oder synthetischer Herkunft, die sich aus mehreren Komponenten zusammensetzen kann: Tenside und Detergenzien aller Art, beispielsweise ionische wie AOT (Natrium-(2-ethylhexyl)-sulfosuccinat), CTAB (Cetyl-trimethyl-ammoniumbromid); nicht ionische wie Brij, Tween 85, Triton X-100; zwitterionische wie Lecithine, Betaine, Aminoxide, usw.
4) mindestens eine *Erkennungskomponente,* wobei, falls die Erkennungskomponente schon eine hinreichende Menge H₂O enthält, wie z.B. die Hydratationshülle eines Enzyms, diese in gewissen Fällen bereits als hydrophile Phase betrachtet werden kann.

Erkennungskomponenten können synthetisch und teilsynthetisch hergestellt und durch Modellierung geplant werden, oder in der Natur vorhanden sein. Beispiele dafür sind: Enzyme, synthetische und genmanipulierte Enzyme; Antikörper, Antikörperfragmente, synthetische und genmanipulierte Antikörper; Rezeptoren und deren Hybride, synthetische und genmanipulierte Rezeptoren; Peptide, Kohlenhydrate, Lektine, Lipide und deren Hybride, synthetische Liganden, Komplex-, Clathrat- und Assoziatbildner, Carrier, Chromoionophoren, Ionophoren, Redoxfarbstoffe, pH-Indikatoren und Makromoleküle, Organe, Organellen, Mikroorganismen.

Je nachdem, ob die hydrophile oder die lipophile Phase im Überschuss vorliegt, werden bei einer Tensidkonzentration grösser als CMC (Critical Micelle Concentration) normale Mizellen oder Umkehrmizellen gebildet. Die Grösse der Umkehrmizellen wird durch das molare Verhältnis Wₒ von Wasser zu Tensid bestimmt (Wₒ = [H₂O] / [Tensid]) . Bei zunehmendem Wassergehalt spricht man bekanntlich auch von Wasser-in-Öl Mikroemulsionen ('w/o microemulsions'); und analog, falls die polare Phase überwiegt, von Öl-in-Wasser Mikroemulsionen ('o/w microemulsions') .

### 1. Bildung des mizellaren Erkennungssystems

Die Bildung des mizellaren Erkennungssystems (MES) kann auf verschiedenen Wegen erfolgen, und zwar durch beliebige Kombination der Mischung der vier genannten Komponenten. Aus praktischen Gründen werden meistens folgende Prozeduren bevorzugt durchgeführt, die stellvertretend für eine Vielzahl von möglichen, aber nicht näher beschriebener Prozeduren aufgeführt werden:

### Prozedur A

a) Bildung einer ersten Mischung aus den Komponenten 1 und 3, nämlich aus einer lipophilen Phase und einer grenzflächenaktiven Substanz.
b) Bildung einer zweiten Mischung aus den Komponenten 2 und 4, nämlich aus einer hydrophilen Phase und einer Erkennungskomponente.
c) Zusammenfügen der ersten und der zweiten Mischung in einem bestimmten Verhältnis Wₒ = [H₂O]/[Tensid] und anschliessendes Durchmischen.
d) Vortex (Zentrifugal-Vibrationsmischer) bis zur Mizellenbildung, die daran erkennbar ist, dass eine klare homogene Phase erhalten wird.

### Prozedur B

a) Bildung einer ersten Mischung aus den Komponenten 1, 2 und 3, nämlich aus einer lipophilen Phase, aus einer hydrophilen Phase und einer grenzflächenaktiven Substanz, in einem bestimmten Verhältnis Wₒ.
b) Vortex (Zentrifugal-Vibrationsmischer) bis zur Mizellenbildung, die daran erkennbar ist, dass eine klare homogene Phase erhalten wird.
c) Bilden einer zweiten Mischung aus der ersten Mischung, welche als klare und homogene Phase vorliegt, durch Zugabe der Komponente 4, nämlich einer Erkennungskomponente.
d) Rühren der zweiten Mischung bis zur homogenen Verteilung.

### Prozedur C

a) Bildung einer ersten Mischung aus den Komponenten 1, 3 und 4, nämlich aus einer lipophilen Phase, aus einer grenzflächenaktiven Substanz und aus einer Erkennungskomponente, die zu einem Erkennungsschritt 2. Art führt.
b) Bildung einer zweiten Mischung aus den Komponenten 2 und 4, nämlich aus einer hydrophilen Phase und einer Erkennungskomponente, die zu einem Erkennungsschritt 1. Art führt.
c) Zusammenfügen der ersten und der zweiten Mischung in einem bestimmten Verhältnis Wₒ = [H₂O]/[Tensid] und anschliessendes Durchmischen.
d) Vortex (Zentrifugal-Vibrationsmischer) bis zur Mizellenbildung, die daran erkennbar ist, dass eine klare homogene Phase erhalten wird.

### Prozedur D

a) Bildung einer ersten Mischung aus den Komponenten 1, 3 und 4, nämlich aus einer lipophilen Phase, aus einer grenzflächenaktiven Substanz und aus zwei Erkennungskomponenten, die beide zu einem Erkennungsschritt 2. Art führen.
b) Bildung einer zweiten Mischung aus den Komponenten 2 und 4, nämlich aus einer hydrophilen Phase und einer Erkennungskomponente, die zu einem Erkennungsschritt 1. Art führt.
c) Zusammenfügen der ersten und der zweiten Mischung in einem bestimmten Verhältnis Wₒ = [H₂O]/[Tensid] und anschliessendes Durchmischen.
d) Vortex (Zentrifugal-Vibrationsmischer) bis zur Mizellenbildung, die daran erkennbar ist, dass eine klare homogene Phase erhalten wird.

Falls mehrere mizellare Erkennungssysteme zur Bildung vorgesehen sind, können diese entweder mit der gleichen oder mit einer beliebigen Kombination von Prozeduren hergestellt werden.

### 2. Einbringen des mizellaren Erkennungssystems in eine Schichtstruktur

Das Einbringen des mizellaren Erkennungssystems in eine Schichtstruktur kann auf verschiedene Weise erfolgen:
2.1 Ein *viskositätsbestimmender Hilfsstoff* wird in der lipophilen Phase des mizellaren Erkennungssystems gelöst, um die erforderlichen physikalischen Eigenschaften der zu erstellenden Schicht zu erbringen. In Frage kommen Polymere und Copolymere sowohl natürlicher wie synthetischer Art, wie Polyvinylchlorid (PVC) und seine Derivate (PVC-OH, PVC-CH₂OH, PVC-NH₂, PVC-NHR, PVC-COOH), Polystyrol, Poly(styrol-butadien), Polyurethane, Polyamide, Acrylate, Polymethacrylate, Poly-(vinylisobutylether), Organosilikate und Silikone. In anderen Fällen können auch 'in situ'-Polymerisationen vorgenommen, wie z.B. Photopolymerisationen, Elektropolymerisationen, oder Polymerisationen, die von einem Monomeren und einem Initator ausgehen. Dies erfolgt beispielsweise mit Silanen, Siloxanen, Acrylaten, Urethanen. Im weiteren kommen Geliermittel wie Gelatine, Pektin, Bentonit und ähnliche mehr in Frage.
2.2 Um im weiteren die erforderlichen chemischen und physikalischen Eigenschaften der Schicht zu erhalten, können *Weichmacher* als Lösungsmittel der Mischung (2.1) beigefügt werden. Weichmacher sind in der Regel niedermolekulare, bei Raumtemperatur nichtflüchtige, flüssige organische Substanzen wie Ester von organischen oder anorganischen Säuren, Alkohole, Ether, Ketone, Sulfonamide und halogenierte Kohlenwasserstoffe, u.a.m.. Typische Beispiele sind Bis(2-ethylhexyl)-sebacate (DOS), ortho-Nitrophenyloctylether (o-NPOE) und Derivate, Bis(2-ethylhexyl)phtalat oder -adipat, Bis(1-butyl-pentyl)adipat (BBPA).
2.3 Zur Mischung (2.2) können weitere *Hilfsstoffe* und *Additive* zum Aufbau der Schicht zugefügt werden. Dies ist beispielsweise erforderlich für die Wahrung der Elektroneutralität, für die Katalyse oder die Pufferung, oder aber zum Erzielen einer bestimmten Löslichkeit.
2.4 Die auf diese Weise erhaltene Mischung (2.3) aus dem mizellaren Erkennungssystem gemäss den Verfahrensschritten 2.1 - 2.3 wird *Cocktail* genannt. Ein derartiger Cocktail zeichnet sich durch eine geeignete Viskosität aus, die eine optimale Weiterverarbeitung, bzw. ein optimales Handling, ermöglicht. Die Viskosität ist meist recht niedrig. Sollte dies nicht der Fall sein, so wird durch Zufügen eines leichtflüchtigen Lösungsmittels die optimale Viskosität für das Auftragen des Cocktails auf einem Träger erzielt.
2.5 Der Cocktail wird auf einem geeigneten Träger aufgetragen, was in bekannterweise mit sogenannten Spin-on-Geräten oder durch Auftropfen erfolgt. Durch das vollständige Verdampfen des organischen Lösungsmittels wird nach anschliessender Trocknung eine *Schicht* hergestellt, in welcher das mizellare Erkennungssystem vorliegt. Bemerkenswert ist, dass in dieser einen einzigen Schicht sämtliche Komponenten für den Erkennungsschritt 1. Art und den Transduktionsschritt nebeneinander verteilt vorliegen, ohne dass dabei die Haltbarkeit der Erkennungskomponente beeinträchtigt wird.

Falls mehrere mizellare Erkennungssysteme vorliegen, können diese entweder vereinigt in einem einzigen Cocktail zu einer einzigen Schicht führen, oder die mizellaren Erkennungssysteme werden in verschiedenen Cocktails zu mehreren aufeinanderfolgenden einzigen Schichten verarbeitet.
2.6 Für das Design der Schichtstruktur können zu der unter 2.5 erhaltenen Schicht, bzw. erhaltenen Schichten zusätzliche Hilfsschichten zugefügt werden, wie beispielsweise Tecoflex, Nafion, usw.. Diese Hilfsschichten dienen der verbesserten Bioverträglichkeit, der Erhöhung der mechanischen Festigkeit, der verbesserten Lebensdauer oder als Diffusionsbarriere, bzw. ganz allgemein als Pufferschicht wie z.B. für Substrate, Produkte oder H⁺-Aktivität. Die Schichtstruktur kann auch mit mehreren Trägern, die unterschiedliche Funktion haben können, in Kontakt stehen.

### 3. Kontaktieren der Substanz mit der Schichtstruktur

Die im Schritt 2 erhaltene Schichtstruktur wird mit der Substanz in Kontakt gebracht. Die Substanz kann sich in einer flüssigen oder gasförmigen Phase befinden; sie kann direkt im Specimen oder in einer aufbereiteten Probe bestimmt werden. Beispiele für ungeladene Substanzen wie Harnstoff, Creatinin, Tenside, Alkohole, Aceton, Glucose, Kohlenhydrate, Glycole, Silikone, Polymere, Farbstoffe, Pestizide, Herbizide, Akarizide; Hormone wie Glukagon, Insulin, Serotonin, Adrenalin, Steroide; Neurotransmittoren, wie Serotonin, Adrenalin, Acetylcholin, DOPA; Gase, aromatische und aliphatische Kohlenwasserstoffe, und metallorganische Verbindungen. Neutrale Moleküle können auch bestimmte Aminosäuren, Peptide und Proteine sein. Typische Ionen sind H⁺, Li^{+,} K⁺, Na⁺, Rb⁺, Cs^{+,} Ba²⁺, Mg²⁺, Ca²⁺, Sr²⁺, Cu²⁺, NH₄ ⁺, Cl⁻, ClO₄⁻, H₂PO₄⁻, HPO₄²⁻, PO₄₋³⁻, HAsO₄²⁻, H₂AsO₄⁻, AsO₄³⁻, SeO₃²⁻, HSeO₃⁻, SeO₄²⁻, SO₃²⁻, HSO₃⁻, SCN⁻, CN⁻, WO₄²⁻. Andere geladene Verbindungen sind Silicate, organische Moleküle wie Sulfonate, Phosphonate, metallorganische Verbindungen und Metallkomplexe, usw.; Metabolite, Substrate und Medikamente wie Heparin, Protamin, Cholin, Phosphatide, organische Säuren, Carboxylate, wie Acetat, Acetacetat, Oxalat, γ- und β-Hydroxybutyrat, δ-Aminolävulinsäure, Salicylate, Phenolate, protonierte Amine, Ammonium-Verbindungen, Alkaloide, optisch aktive und helicale Verbindungen, DNA, RNA, Rezeptoren, Mikroorganismen, Viren, Organellen, Isotope usw.

Überraschenderweise equilibriert das mizellare Erkennungssystem im viskosen System mit der Substanz in der Probe, d.h. das Gleichgewicht, bzw. der 'Steady State' (Fliessgleichgewicht) stellt sich mit hinreichender Geschwindigkeit ein. Damit ist die analytische Verwendbarkeit gewährleistet.

### 4. Erkennungsschritt 1. Art

Beim Kontaktieren wird die zu bestimmende Substanz in die Schichtstruktur extrahiert und reagiert in der einzigen Schicht mit der Erkennungskomponente selektiv. Bei gleicher Menge, Konzentration, bzw. Aktivität wird die Substanz um einen Faktor bevorzugt, der in der Regel als log K, Logarithmus des Verteilungskoeffizienten 'über alles' zur Basis 10 (overall distribution) angegeben wird, während andere Substanzen um diesen Faktor diskriminiert werden. Dabei kann die Substanz je nach Art der Wechselwirkung (vgl. 3.5, bzw. 3.6) in weitere Produkte umgesetzt werden. Die Wechselwirkung kann durch Komplexierung, Redox-Prozesse, Additionsreaktionen, Assoziation und Dissoziation, Bildung von Einschlussverbindungen (Clathraten), Hydrolyse, Gruppen-, Protonen- und Elektronen-Transfer-Reaktionen, Ionenaustausch, usw. erfolgen.

### 5. Transduktionsschritt

Als Folge der molekularen Erkennung im Schritt 4 wird eine Messgrösse erzeugt. Dieses kann entweder direkt quantifiziert werden oder durch Kopplung mit einem oder mehreren Erkennungsschritten 2. Art in eine messbare Grösse umgewandelt werden. Der Transduktionschritt kann durch Photonen-, Elektronen-, Massentransfer, durch Elektronenübergänge, über Anregungszustände vorgenommen werden. Dieses Signal kann photometrisch, potentiometrisch, amperometrisch, voltammetrisch, polarographisch, coulometrisch, konduktometrisch, durch Zählungen, durch Messung von Zerfallszeiten, der Fluoreszenzlebensdauer, der Reaktionsgeschwindigkeit, von Abklingzeiten, von pH-Änderungen, usw. erfasst werden. Änderungen der Viskosität, der Kraft, der Masse, der Dispersion, der Frequenz, der dielektrischen Eigenschaften (permittivity), der Polarisationsebene, der Rotationsgeschwindigkeit, des Winkels können auch zur Transduktion verwendet werden.

Innerhalb dieses Transduktionschrittes können *Erkennungsschritte 2. Art* vorkommen. Letztere sind die Folge des Erkennungsschrittes 1. Art. Bei Erkennungsschritten 2. Art finden einerseits Wechselwirkungen zwischen den *Produkten* des Erkennungsschrittes 1. Art und weiterer vorliegender *Erkennungskomponenten* statt, oder andererseits induziert der Erkennungsschritt 1. Art einen Erkennungsschritt 2. Art.

Während die Erkennungskomponenten, die zu einem Erkennungsschritt 1. Art führen, einen selektiven Charakter haben müssen, ist dies für die Erkennungskomponenten, die zu einem Erkennungsschritt 2. Art führen, nicht zwingend notwendig.

Falls mehrere mizellare Erkennungssysteme vorliegen, hat jedes mizellare Erkennungssystem einen Transduktionsschritt zur Folge, wobei die Transduktionsschritte untereinander nicht zwingend verschieden sind.

### 6. Messgrösse

Die im Transduktionsschritt 5 erhaltene Messgrösse kann ganz verschiedener Natur sein: Absorptions-, Strom-, Potential-, Leitfähigkeits-, Brechungindex-, Temperatur-, Viskositäts-, Dispersions-, Polarisations- und Frequenzsänderung, u.a.m.. Aus dieser Messgrösse wird direkt oder nach mathematischen Umwandlungen die Information über die Substanz gewonnen: Menge, Konzentration, Aktivität und dergleichen.

Die Messgrösse liefert somit Information über die zu bestimmende Substanz, welche beispielsweise auf einem Anzeigeelement sichtbar gemacht werden kann, oder aber in einer analogen oder digitalen Form für eine weitergehende Verarbeitung verfügbar gehalten wird. So kann beispielsweise die Messgrösse über einen optischen Wellenleiter oder ein Halbleiterelement weitergeführt, bzw. weiterverarbeitet werden, was hier nicht näher ausgeführt wird. Es können auch mehrere Messgrössen gleichzeitig als Folge des Transsduktionsschrittes erhalten werden. Dies kann durch ein einzelnes mizellares Erkennungssystem, bzw. durch eine Mehrzahl von mizellaren Erkennungssystemen verursacht werden.

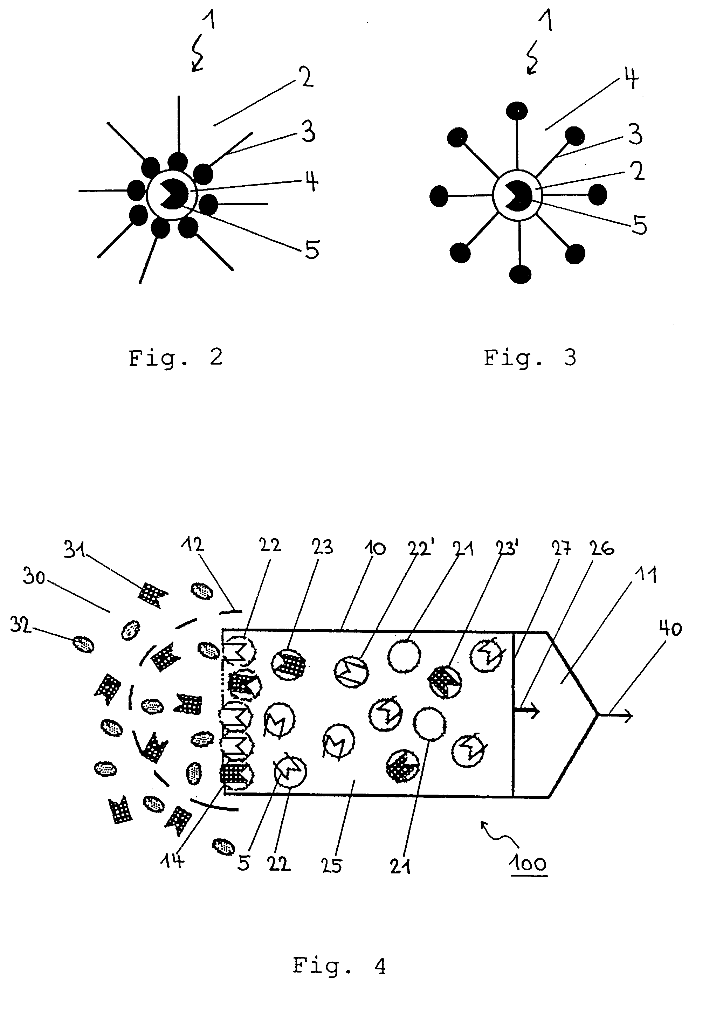

Fig. 2 zeigt eine schematische Darstellung eines mizellaren Erkennungssystems mit Umkehrmizellen. Von einer Schichtstruktur ist hier die Schicht 1 erkennbar, welche aus einer lipophilen Phase 2, einer grenzflächenaktiven Substanz 3, einer hydrophilen Phase 4 und einer Erkennungskomponente 5 besteht. Die Erkennungskomponente 5 liegt in der hydrophilen Phase 4 beispielsweise in wässriger Lösung vor. Die grenzflächenaktive Substanz 3 umgibt die hydrophile Phase 4 derart, dass die grenzflächenaktive Substanz den Bereich der hydrophilen Phase als Umkehrmizelle umschliesst und auf diese Weise die in der hydrophilen Phase gelöste Erkennungskomponente gegen die unerwünschten Einflüsse der lipophilen Phase 2 wirkungsvoll abschirmt. Dabei kann die Erkennungskomponente auch teilweise in die benachbarte Phase ragen. Durch diese Art der Abschirmung wird beispielsweise eine Denaturierung der Erkennungskomponente umgangen und dadurch die Lebensdauer, die Löslichkeit und die Verteilung oder Dispersion der Erkennungskomponente verbessert. Ein derartiges mizellares Erkennungssystem kann auch Umkehrmizellen ohne Erkennungskomponenten, sogenannte leere Mizellen, aufweisen.

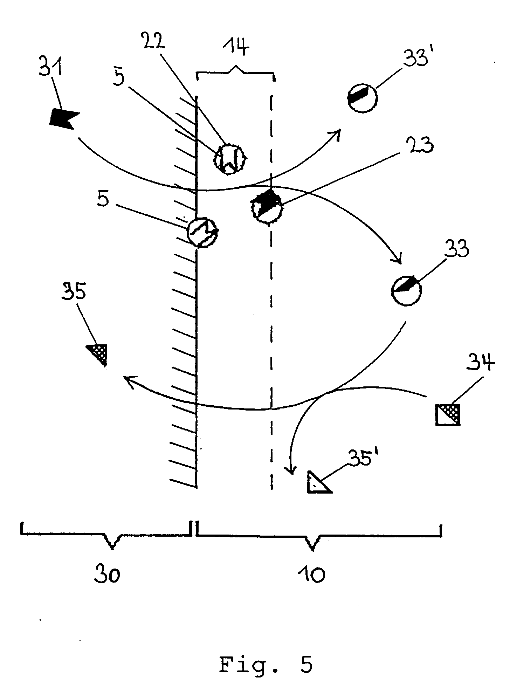

Fig. 3 zeigt eine schematische Darstellung eines mizellaren Erkennungsystems mit normaler Mizellanordnung. Von einer Schichtstruktur ist hier eine Schicht 1 erkennbar, welche aus einer hydrophilen Phase 4, einer grenzflächenaktiven Substanz 3, einer lipophilen Phase 2 und einer Erkennungskomponente 5 besteht. Die Erkennungskomponente 5 liegt in der lipophilen Phase 2 beispielsweise in einer Bis(2-ethylhexyl)-sebacate (DOS) - Lösung vor. Die grenzflächenaktive Substanz 3 umgibt die lipophile Phase 2 derart, dass die grenzflächenaktive Substanz den Bereich der lipophilen Phase als normale Mizellen umschliesst und auf diese Weise die in der lipophilen Phase gelöste Erkennungskomponente gegen die unerwünschten Einflüsse der hydrophilen Phase 4 wirkungsvoll abschirmt. Dabei kann die Erkennungskomponente auch teilweise in die benachbarte Phase ragen. Ein derartiges mizellares Erkennungssystem kann auch Mizellen ohne Erkennungskomponenten, sogenannte leere Mizellen, aufweisen.

Fig. 4 zeigt eine schematische Darstellung des eingebrachten mizellaren Erkennungssystems als Schicht einer Schichtstruktur gemäss Prozedur A. Eine Schicht 10 ist auf der einen Seite umgeben von einem Träger 11 und auf der anderen Seite von einer Hilfsschicht 12. Die Schicht 10, der Träger 11 und die Hilfsschicht 12 bilden gemeinsam eine Schichtstruktur 100. Die Schicht 10 besteht aus leeren Mizellen 21, aus den unbesetzten Mizellen 22 und 22' und aus den besetzten Mizellen 23 und 23', sowie aus der nahezu vollständig vom flüchtigen Lösungsmittel befreiten Phase 25, welche die Mizellen umgibt. Eine Probe 30, bestehend aus einer Substanz 31 und weiteren Komponenten, von denen nur eine diskriminierte Substanz 32 abgebildet ist, wird mit der Schichtstruktur 100 in Kontakt gebracht, wobei sich die Probe 30 auf der dem Träger 11 gegenüberliegenden Seite der Schicht 10 befindet. Die Hilfsschicht 12 befindet sich ebenfalls auf der dem Träger 11 gegenüberliegenden Seite der Schicht 10 und ist weitestgehend durchlässig oder für Probenkomponenten gezielt semipermeabel.

Die leeren Mizellen 21 der Schicht 10 sind solche, welche beim Aufbau der Schicht keine Erkennungskomponente 5 enthalten, wohl aber Teil des mizellaren Systems sind.

Die unbesetzten Mizellen 22 und 22' enthalten eine Erkennungskomponente 5 und stellen somit eine Komponente des mizellaren Erkennungssystems dar. Während die unbesetzte Mizelle 22 die Erkennungskomponente 5 praktisch umschliesst, ragt die Erkennungskomponente 5 in der unbesetzten Mizelle 22' teilweise in die benachbarte Phase. Die unbesetzten Mizellen 22 und 22' sind Teil des mizellaren Erkennungssystems vor der Wechselwirkung zwischen der Substanz 31 und der Erkennungskomponente 5.

Die besetzten Mizellen 23 sind Teil des mizellaren Erkennungssystems nach der Wechselwirkung zwischen der Substanz 31 und der Erkennungskomponente 5.

Die Schicht 10 weist an derjenigen Stelle, welche der Probe 30 ausgesetzt ist, einen reaktiven Bereich 14 auf, in welchem die leeren Mizellen 21, die unbesetzten Mizellen 22 und die besetzten Mizellen 23 vorliegen.

Im weiteren wird nun die Funktionsweise des mizellaren Erkennungssystems erläutert. Beim Kontaktieren der Substanz 31, welche Bestandteil der Probe 30 ist, mit dem reaktiven Bereich 14 wird die Substanz selektiv durch das mizellare Erkennungssystem erkannt, was mit einem Erkennungsschritt 1. Art bezeichnet wird und mit den Mizellen 23 dargestellt ist. Die diskriminierte Substanz 32 wird vom mizellaren Erkennungssystem nicht oder kaum erkannt, wodurch sich eine entsprechend hohe Selektivität des Verfahrens ergibt. Die Probe 31 und die Schicht 10 sind grundsätzlich zwei nicht-mischbare Phasen. Die Substanz 31 aus der Probe 30 verteilt sich in Abhängigkeit der Zeit in den besetzten Mizellen 23 und 23' homogen über die ganze Schicht 10. Dies gilt ebenfalls für leere und unbesetzte, sowie für andere Schichtkomponenten. Dies erfolgt in der Regel schnell , da die Schicht 10 dünn ausgelegt und typischerweise kleiner 50 µm ist. Nachdem im Erkennungsschritt 1. Art die molekulare Erkennung erfolgt ist, folgt nun der Transduktionsschritt, bei welchem eine Messgrösse erzeugt wird. Durch die Veränderung der Anzahl besetzter Mizellen bildet sich die Messgrösse als Änderung einer physikalischen Eigenschaft, beispielsweise des Brechungsindex, der Temperatur und dergleichen (vgl. Schritt 6). An einer Grenzfläche 27, welche sich zwischen der Schicht 10 und dem Träger 11 befindet, wird beispielsweise eine Messgrösse erzeugt, was mit dem Pfeil 26 angedeutet ist. Die Messgrösse stellt ein optisches Signal dar, falls der Träger als optischer Wellenleiter ausgebildet ist. Dadurch wird die Messgrösse in eine Information 40 übergeführt, welche als solche weiterverarbeitet, bzw. weiterverwendet werden kann.

Fig. 5 zeigt eine schematische Darstellung eines Erkennungsschrittes 1. Art gekoppelt an einen Erkennungsschritt 2. Art in einer Schicht. Die Substanz 31 der Probe 30 verbindet sich mit der Erkennungskomponente 5 über einen Erkennungsschritt 1. Art zur besetzten Mizelle 23. Dies führt zu den Produkten 33 und 33'. Die Erkennungskomponente 5 ist Bestandteil des mizellaren Erkennungssystems und befindet sich in der unbesetzten Mizelle 22 im reaktiven Bereich 14 der Schicht 10. In einem Erkennungsschritt 2. Art löst das Produkt 33 in einer weiteren Reaktion die Transformation einer weiteren Erkennungskomponente 34 aus, wobei zweite Produkte 35 und 35' entstehen. Die Erkennungskomponente 34 kann in der Schicht 10 beliebig verteilt sein, einschliesslich im reaktiven Bereich 14, nämlich beispielsweise in der Mizelle, oder in der die Mizelle umgebenden Phase, d.h. entweder in der lipophilen oder in der hydrophilen Phase. Das zweite Produkt 35 kann mindestens teilweise wieder in die Probe 30 austreten, bzw. von der Probe 30 aus der Schicht 10 ausgewaschen werden, oder als zweites Produkt 35' sich mindestens teilweise in der Schicht 10 in einer leeren oder jeder anderen Mizelle, oder irgendwo in der Schicht verteilen. Der Erkennungsschritt 2. Art ist Teil der Transduktion und führt zur Bildung der Messgrösse.

### 5. Beispiele

Beispiel 1 beschreibt eine Schichtstruktur einer mizellaren Harnstoff-Biooptode.

Als Komponenten des mizellaren Erkennungssystems wurden Cyclohexanon (lipophile Phase), AOT (grenzflächenaktive Substanz), H₂O (hydrophile Phase) und Urease (Erkennungskomponente) gewählt.

Die Bildung des mizellaren Erkennungssystems wurde nach der Prozedur D durchgeführt (Schritt 1) :
a) Bildung einer ersten Mischung: 1.7 ml einer 25 mM AOT Lösung in Cyclohexanon. Weitere Zugabe von 1.8 mg Nonactin (Ionophor als erste Erkennungskomponente 2. Art), 1.3 mg Chromoionophor ETH 5350 (Indikator als zweite Erkennungskomponente 2. Art).
b) Bildung einer zweiten Mischung: wässrige Lösung von Urease, wobei 20 mg Urease in 100 µl Wasser gelöst wurden, was 1'600 Einheiten entsprach.
c) 60 µl der wässrigen Urease-Lösung der zweiten Mischung wurden zur ersten Mischung gegeben, so dass ein Wₒ-Wert von 78.4 resultierte.
d) Nach Vortex (Zentrifugal-Vibrationsmischer) wurde eine homogene Mischung erhalten.

Das Einbringen des mizellaren Erkennungssystems in die Schichtstruktur wurde wie folgt realisiert (Schritt 2) : 88 mg PVC (Polymer) und 170 mg ortho-NPOE (ortho-Nitrophenyl-octylether, Weichmacher) und 2.5 mg NaTFPB (Natrium-tetrakis (3,5-bis(trifluoromethyl)-phenyl)borat, Additiv) wurden zu 1.7 ml der homogenen Mischung zugegeben. Nach Rühren und kurz Erwärmen auf 30 °C wurde eine klare visköse Lösung, der sogenannte Cocktail, erhalten.
0.3 ml Cocktail wurden auf einem Quarzträger in einem Spin-on-Gerät (Seiler, K., Simon, W., Anal. Chim. Acta, 266 (1992) 74) aufgetragen. Beim Verdampfen des organischen Lösungsmittels entstand eine Schicht mit einer Dicke von 2 bis 4 µm. Die Schicht wurde während vier Stunden bei Raumtemperatur getrocknet und bildete so gleichzeitig auch die Schichtstruktur, da keine weiteren Hilfsschichten zur Bildung der Schichtstruktur benötigt wurden.

Kontaktieren der Substanz mit der Schichtstruktur (Schritt 3):
Jeweils zwei mit der Schichtstruktur belegte Quarzträger wurden in einer Durchflusszelle eines Doppelstrahl-Spektrophotometers (Uvikon 942) fixiert, die auf einer bei 25 °C thermostatisierten Grundplatte im Messkanal montiert wurde. Die Schichtstruktur wurde mit einer 30 mM Natriumphosphat Pufferlösung mit pH 7.1 konditioniert. Es wurden gepufferte Harnstoff-Lösungen, in denen der Harnstoff die zu bestimmende Substanz darstellte, in verschiedenen Konzentrationen gemessen. Die Änderung der Extinktion (Messgrösse) beim Absorptionsmaximum der protonierten Form des Chromoionophor ETH 5350 wurde bei 652 nm verfolgt und ergab die nachstehenden Messwerte:

| | | | | |
|---|---|---|---|---|
| [Harnstoff] | 0 | 0,001 M | 0,01 M | 0,1 M |
| Extinktion (652 nm) | 0,136 | 0,118 | 0,080 | 0,052 |

Im weiteren wird die Funktionsweise der mizellaren Harnstoff-Biooptode beschrieben:

Die selektive Erkennung von Harnstoff durch das Enzym Urease in einem mizellaren Erkennungssystem (Schritt 4) wird mit einem optischen Transduktionsschritt (Schritt 5) gekoppelt (Optode). Das Ansprechverhalten eines derartigen Sensors beruht auf einem Ionenaustausch-Gleichgewichts-Mechanismus, der im folgenden erläutert wird. Bei der enzymatisch katalysierten Hydrolyse von Harnstoff (vgl. Reaktion 1) entstehen, entsprechend dem Wassergehalt der Mizelle und dem Arbeits-pH, NH₄⁺-Ionen, bzw. Ammoniak (NH₃), die in einem Erkennungsschritt 2. Art vom Ionophor Nonactin (L) komplexiert werden. Diese Komplexierung bewirkt seinerseits die Deprotonierung des Indikators (H⁺-selektiven Chromoionophor ETH 5350) nach Reaktion 2: 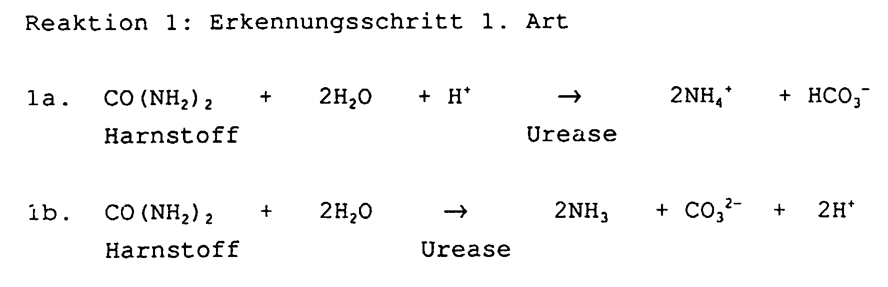 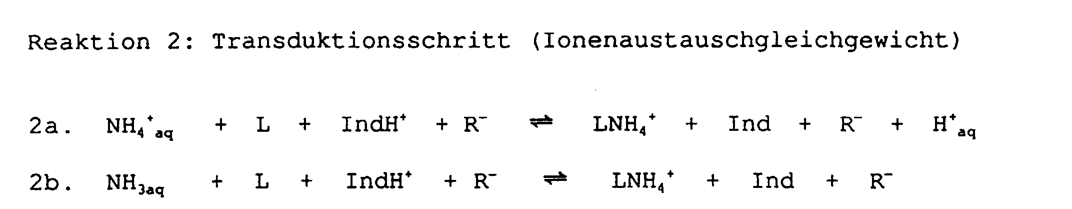 Die totale Kationenkonzentration in der elektrisch neutralen Schicht wird durch das ionische Additiv R⁻ festgelegt. Die beim Transduktionsschritt erhaltene Messgrösse (Schritt 6) ist die Deprotonierung des Indikators und die damit verbundene Extinktionsänderung, über welche die Information über die Substanz, bzw. die Harnstoff-Aktivität, ermittelt wird. Zum Ausschluss störender Kationen kann die Schicht mit einer kationischen Hilfsschicht überzogen werden. Dabei wird eine Schichtstruktur gebildet.

Beispiel 2 beschreibt eine Schichtstruktur einer mizellaren α-Chymotrypsin-Optode.

Als Komponenten des mizellaren Erkennungssystems wurden Cyclohexan (lipophile Phase), AOT (grenzflächenaktive Substanz), H₂O (hydrophile Phase) und α-Chymotrypsin (Erkennungskomponente) gewählt.

Die Bildung des mizellaren Erkennungssystems wurde nach der Prozedur A durchgeführt (Schritt 1):
a) Bildung einer ersten Mischung: 1.5 ml einer 25 mM AOT Lösung in Cyclohexan.
b) Bildung einer zweiten Mischung: wässrige Lösung von α-Chymotrypsin, wobei 18 mg α-Chymotrypsin in 100 µl Wasser gelöst wurden.
c) 20 µl der wässrigen α-Chymotrypsin-Lösung der zweiten Mischung wurden zur ersten Mischung gegeben, so dass ein Wₒ-Wert von 25.0 resultierte.
d) Nach Vortex (Zentrifugal-Vibrationsmischer) wurde eine homogene Mischung erhalten.

Das Einbringen des mizellaren Erkennungssystems in die Schichtstruktur wurde wie folgt realisiert (Schritt 2): 120 mg PSB (Polystyrolbutadien, Polymer) und 87 mg BBPA (Bis(1-butylpentyl)adipat, Weichmacher) wurden zu 1.5 ml der homogenen Mischung zugegeben. Nach Rühren wurde eine klare visköse Lösung, der sogenannte Cocktail, erhalten.
0.3 ml Cocktail wurden auf einem Quarzträger in einem Spin-on-Gerät (Seiler, K., Simon, W., Anal. Chim. Acta, 266 (1992) 74) aufgetragen. Beim Verdampfen des Cyclohexans entstand eine Schicht mit einer Dicke von 2 bis 5 µm. Die Schicht wurde während drei Stunden bei Raumtemperatur getrocknet und bildete so gleichzeitig auch die Schichtstruktur, da keine weiteren Hilfsschichten zur Bildung der Schichtstruktur benötigt wurden.

Kontaktieren der Substanz mit der Schichtstruktur (Schritt 3):
Jeweils zwei mit der Schichtstruktur belegte Quarzträger wurden in einer Durchflusszelle eines Doppelstrahl-Spektrophotometers (Uvikon 942) fixiert, die auf einer bei 25 °C thermostatisierten Grundplatte im Messkanal montiert wurde. Die Schichtstruktur wurde mit einer 50 mM Tris-Pufferlösung mit pH 8.0 konditioniert. Es wurden gepufferte Ac-Trp-ONp-Lösungen (Acetyl-L-tryptophan-p-nitrophenylester), in denen Ac-Trp-ONp die zu bestimmende Substanz darstellte, in verschiedenen Konzentrationen gemessen. Die Änderung der Extinktion (Messgrösse) bei 400 nm wurde verfolgt.

Im weiteren wird die Funktionsweise der mizellaren α-Chymotrypsin-Biooptode beschrieben:

Die selektive Erkennung von Ac-Trp-ONp (Acetyl-L-tryptophan-p-nitrophenylester) durch das Enzym α-Chymotrypsin in einem mizellaren Erkennungssystem (Schritt 4) wird direkt spektrophotometrisch erfasst. In diesem Fall sind keine weiteren Erkennungskomponenten notwendig. Bei der enzymatisch katalysierten Hydrolyse von Ac-Trp-ONp entsteht das Produkt p-Nitrophenol (p-Np), welches bei 400 nm direkt spektrophotometrisch erfasst wird (Schritt 5).

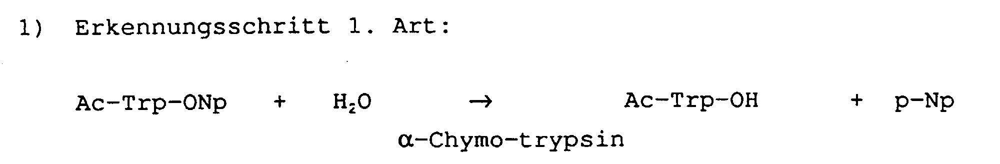

### 2) Transduktionsschritt:

Der Transduktionsschritt erfolgt durch die direkte spektrophotometrische Erfassung des entstandenen Produktes p-Nitrophenol (p-Np) bei 400 nm.

Die beim Transduktionsschritt erhaltene Messgrösse (Schritt 6) ist die Extinktionsänderung bei 400 nm, über welche die Information über die Substanz, bzw. die Konzentration von Ac-Trp-ONp, ermittelt wird.

### Beispiel 3 beschreibt eine Schichtstruktur eines mizellaren Immunosensors.

Als Komponenten des mizellaren Erkennungssystems wurden Cyclohexan (lipophile Phase), AOT (grenzflächenaktive Substanz), H₂O (hydrophile Phase) und Anti-Maus IgG (Erkennungskomponente) gewählt.

Die Bildung des mizellaren Erkennungssystems wurde nach der Prozedur A durchgeführt (Schritt 1):
a) Bildung einer ersten Mischung: 1.5 ml einer 25 mM AOT Lösung in Cyclohexan.
b) Bildung einer zweiten Mischung: wässrige Lösung von Anti-Maus IgG, wobei 1.8 mg Anti-Maus IgG in 1000 µl Wasser gelöst wurden.
c) 23 µl der zweiten Mischung wurden zur ersten Mischung gegeben, wobei ein Wₒ-Wert von 30 erhalten wurde.
d) Nach Vortex (Zentrifugal-Vibrationsmischer) wurde eine homogene Mischung erhalten.

Das Einbringen des mizellaren Erkennungssystems in die Schichtstruktur wurde wie folgt realisiert (Schritt 2):
120 mg PSB (Polystyrolbutadien, Polymer) und 87 mg BBPA (Bis(1-butylpentyl)adipat, Weichmacher) wurden zu 1.5 ml der homogenen Mischung zugegeben. Nach Rühren wurde eine klare visköse Lösung, der sogenannte Cocktail, erhalten.
0.3 ml Cocktail wurden auf einer Seite eines Saphir-Einkristalles in einem Spin-on-Gerät (Seiler, K., Simon, W., Anal. Chim. Acta, 266 (1992) 74) aufgetragen. Der Saphir-Einkristall, der als optischer Wellenleiter diente, wies die Masse 5 cm x 2 cm x 1 mm auf, dessen Winkel an den Kanten 60°, bzw. 120° betrugen. Beim Verdampfen des Cyclohexans entstand eine Schicht mit einer Dicke von 2 bis 5 µm. Die Schicht wurde während vier Stunden bei Raumtemperatur getrocknet und bildete so gleichzeitig auch die Schichtstruktur, da keine weiteren Hilfsschichten zur Bildung der Schichtstruktur benötigt wurden.

### Kontaktieren der Substanz mit der Schichtstruktur (Schritt 3) :

Die Schichtstruktur auf dem Kristall wurde in eine speziell angefertigte ATR-Messzelle eingespannt. Die Messungen wurden in einem Zweistrahl-Photometer gegen Luft als Referenz durchgeführt. Die Schichtstruktur wurde mit einer 50 mM Tris-Pufferlösung mit pH 8.0 konditioniert. Danach wurden gepufferte Lösungen des Proteins Maus IgG, welches die zu bestimmende Substanz darstellte, in verschiedenen Konzentrationen in Kontakt mit der Schichtstruktur gebracht. Die Änderung des Brechungsindex (Messgrösse) der Schichtstruktur wurde gemessen.

Im weiteren wird die Funktionsweise des mizellaren Immunosensors beschrieben:

Die selektive Erkennung des Proteins Maus-IgG (Antigen), welches oft als Modell für die grosse Zahl zellkulturtechnisch hergestellter monoklonaler Antikörper verwendet wird, erfolgt durch das Anti-Maus-IgG (Antikörper) in einem mizellaren Erkennungssystem (Schritt 4). Die Immunreaktion zwischen Antigen-Antikörper wird direkt mit der ATR-Methode (attenuated total reflection) erfasst. Diese Änderung des Brechungsindex (Messgrösse) der Schichtstruktur, bzw. die Geschwindigkeit dieser Änderung, wird nach dem Kontaktieren mit der Antigen-Probe gemessen und ist proportional zur Antigen-Konzentration der Probe.

Selbstverständlich ist dieses Verfahren nicht auf die Bestimmung von Urea, α-Chymotrypsin und Maus-IgG beschränkt. Vielmehr gibt es eine Vielzahl von industriellen Anwendungen, von denen einige im folgenden aufgeführt werden:

Anwendungen für ein derartiges Verfahren und die entsprechenden Schichtstrukturen finden sich in der pharmazeutischen, medizinischen und klinisch-chemischen Analytik und der Umwelttechnik zur Bestimmung von immunchemischen Wechselwirkungen; im Reinraum-Monitoring, in der chemischen Prozesstechnik, der Biotechnologie, in der Abwasser- und Trinkwasserkontrolle.

Im weiteren finden sich Anwendungen in der Agrartechnologie bei der Lagerung von Früchten jeglicher Art, wo der Steuerung der Lageratmosphäre in Kühlhäusern über eine Kontrolle der Gaszusammensetzung eine grosse Bedeutung zukommt. Ebenfalls in der Agrartechnologie oder der Lebensmitteltechnologie von Interesse sind die sogenannten 'Smell Sensors', mit welchen die Aromastofferkennung bei der Klassifizierung von Früchten über deren Dampfphase erfolgen kann.

Anwendungen in der Textilindustrie zeichnen sich bei der Schweissanalyse in Sporttextilien ab.

Anwendungen in der Bautechnik finden sich bei der Bestimmung von Schwermetallen, Chloriden und Mikroorganismen in Beton und Mauerwerken jeglicher Art; in der Raumtechnik bei Lüftungsfragen, bei der Bestimmung des Luftumsatzes und des CO₂-Gehaltes.

Anwendungen in der Waschmittel- und Tensidindustrie zur Kontrolle der Abwasserbelastung mit Tensiden; in der Pharmaforschung, der Arzneimittelproduktion und der Arzneimittelkontrolle für die Bestimmung von Wechselwirkungen zwischen Wirkstoffen und Rezeptoren.

Damit ist eine breite Vielfalt von Anwendungen angedeutet, wobei diese Aufzählung keinesfalls abschliessend ist.

Erfindungswesentlich ist, dass im beschriebenen Verfahren und in den entsprechenden Schichtstrukturen aus einer Erkennungskomponente in geeigneter Art und Weise ein mizellares Erkennungssystem gebildet wird. Dieses mizellare Erkennungssystem wird in eine Schichtstruktur derart eingebracht, dass es in der Schichtstruktur als eine einzige Schicht vorliegt. Dabei zeichnet sich die Erkennungskomponente durch eine verbesserte Lebensdauer aus, und dadurch die ganze Schichtstruktur durch eine verbesserte Haltbarkeit. Besonders vorteilhaft für die Schichtstruktur erweisen sich eine höhere Sensitivität, eine tiefere Nachweisgrenze, eine kurze Ansprechzeit, die mehrmalige, wiederholte Verwendbarkeit und die Robustheit bezüglich der Probeninterferenz.

## Patentansprüche

1. Verfahren zur Bestimmung einer Substanz, **gekennzeichnet dadurch,** daß mindestens ein mizellares Erkennungssystem aus zwei nicht-mischbaren Phasen, aus mindestens einer grenzflächenaktiven Substanz und aus mindestens einer Erkennungskomponente gebildet wird (1), daß das mizellare Erkennungssystem in eine Schichtstruktur eingebracht wird (2), in welcher diese mindestens eine einzige Schicht bildet, daß die Schichtstruktur mit der Substanz in Kontakt gebracht wird (3), daß in dieser einzigen Schicht mindestens ein Erkennungsschritt stattfindet (4), daß dem Erkennungsschritt mindestens ein Transduktionsschritt folgt (5), daß dadurch mindestens eine Meßgröße erzeugt wird (6), und daß über diese Meßgröße mindestens eine Information über die Substanz gewonnen wird.

2. Verfahren nach Anspruch 1, **gekennzeichnet dadurch,** daß die einzige Schicht mittels viskositätsbestimmenden Hilfsstoffen und Weichmachern in ihren physikalischen und chemischen Eigenschaften eingestellt wird.

3. Verfahren nach Anspruch 1 oder 2, **gekennzeichnet dadurch,** daß die Erkennungskomponente mit der zu bestimmenden Substanz wechselwirkt und dadurch die Substanz selektiv erkennt.

4. Verfahren nach einem der Ansprüche 1 bis 3, **gekennzeichnet dadurch,** daß die Schichtstruktur im weiteren aus Hilfsschichten gebildet wird.

5. Verfahren nach einem der Ansprüche 1 bis 4, **gekennzeichnet dadurch,** daß die Substanz Ionen, geladene, ungeladene oder neutrale Moleküle, Isotope, Mikroorganismen, Viren, Organellen, Salze oder Rezeptoren sind.

6. Verfahren nach einem der Ansprüche 1 bis 5, **gekennzeichnet dadurch,** daß die Information über die Substanz als Menge als Konzentration, als Zählresultat oder als Aktivität ermittelt wird.

7. Verfahren nach einem der Ansprüche 1 bis 6, **gekennzeichnet dadurch,** daß die Erkennungskomponente mit der zu bestimmenden Substanz wechselwirkt und danach ein Transduktionsschritt induziert wird.

8. Verfahren nach Anspruch 7, **gekennzeichnet dadurch,** daß vom Produkt der Wechselwirkung zwischen der Erkennungskomponente und der zu bestimmenden Substanz eine weitere nur bedingt selektive Reaktion mit zumindest einer weiteren Erkennungskomponente ausgeht und damit zur Transduktion führt.

9. Anwendung eines Verfahrens nach Anspruch 7 oder 8 in der medizinischen Diagnostik und der klinisch-chemischen Analytik.

10. Anwendung eines Verfahrens nach Anspruch 7 oder 8 in der Umwelttechnik, Bautechnik, Raumtechnik, Reinraumtechnik und der Abwasser- und Trinkwasserkontrolle.

11. Anwendung eines Verfahrens nach Anspruch 7 oder 8 in der Lebensmitteltechnologie, in der Agrartechnologie, in der Biotechnologie und chemischen Prozeßtechnik.

12. Anwendung eines Verfahren nach Anspruch 7 oder 8 in der Pharmaforschung, der Arzneimittelproduktion und der Arzneimittelkontrolle.

13. Anwendung eines Verfahrens nach Anspruch 7 oder 8 in der Textiltechnik und in der Waschmittel- und Tensidindustrie.

14. Schichtstruktur zur Bestimmung einer Substanz, **gekennzeichnet dadurch,** daß in einer Schichtstruktur mindestens ein mizellares Erkennungssystem angeordnet ist, welches aus zwei nicht-mischbaren Phasen, aus mindestens einer grenzflächenaktiven Substanz und aus mindestens einer Erkennungskomponente besteht, daß das mizellare Erkennungssystem in mindestens einer einzigen Schicht homogen verteilt ist und daß mindestens ein Träger vorgesehen ist, der mit der Schichtstruktur in Verbindung steht und den Transduktionsschritt sowie die Gewinnung von Information unterstützt.

15. Schichtstruktur nach Anspruch 14, **gekennzeichnet dadurch,** daß als Erkennungskomponenten Rezeptoren, Antikörper und Enzyme, und deren synthetische und genmanipulierte Hybride und Fragmente alle dieser Komponenten vorgesehen sind.

16. Schichtstruktur nach Anspruch 14 oder 15, **gekennzeichnet dadurch,** daß in der Schichtstruktur neben der mindestens einen einzigen Schicht mit dem mindestens einen mizellaren Erkennungssystem Hilfsschichten angeordnet sind, welche die Bioverträglichkeit und die Lebensdauer verbessern, die mechanische Festigkeit erhöhen, und/oder als Diffusionsbarriere und als Pufferschichten dienen.

17. Schichtstruktur nach einem der Ansprüche 14 bis 16, **gekennzeichnet dadurch,** daß auf einer beliebigen Seite der Schichtstruktur die Probe mit der Substanz und auf einer anderen beliebigen Seite ein Träger angeordnet ist, wobei letzterer der Halterung dient.

18. Schichtstruktur nach einem der Ansprüche 14 bis 17, **gekennzeichnet dadurch,** daß die zwei nicht-mischbaren Phasen aus einer lipophilen Phase geringe Polarität und aus einer hydrophilen Phase erhöhter Polarität bestehen.

## Claims

1. A method for the determination of a substance, characterized in that at least one micellar recognition system is formed from two non-miscible phases, from at least one surface-active substance and from at least one recognition component (1), that the micellar recognition system is incorporated into a layer structure (2) in which it forms at least one single layer, the layer structure is brought into contact with the substance (3), at least one recognition step takes place in said single layer (4), the recognition step is followed by at least one transducing step (5), at least one measurement variable is produced as a consequence (6) and at least one information on the substance is obtained through the said measurement variable.

2. A method as claimed in claim 1, characterized in that the physical and chemical properties of the single layer are adjusted by means of viscosity-controlling additives and plasticizers.

3. A method as claimed in claim 1 or 2, characterized in that the recognition component interacts with the substance to be determined and thus selectively recognizes the substance.

4. A method as claimed in one of the claims 1 to 3, characterized in that the layer structure is further formed by auxiliary layers.

5. A method as claimed in one of the claims 1 to 4, characterized in that the substance is constituted by ions, charged, uncharged or neutral molecules, isotopes, microorganisms, viruses, organelles, salts or receptors.

6. A method as claimed in one of the claims 1 to 5, characterized in that the information on the substance is determined in the form of an amount, a concentration, a counted result or as an activity.

7. A method as claimed in one of the claims 1 to 6, characterized in that the recognition component interacts with the substance to be determined and a transducing step is initiated thereupon.

8. A method as claimed in claim 7, characterized in that the product of the interaction between the recognition component and the substance to be determined triggers at least one further reaction, which is only conditionally selective, with at least one further recognition component and thus leads to the transduction.

9. An application of a method as claimed in claim 7 or 8 in medical diagnosis and clinical-chemical analysis.

10. An application of a method as claimed in claim 7 or 8 in environmental technology, structural engineering, space technology, clean room technology, monitoring of drinking and waste water.

11. An application of a method as claimed in claim 7 or 8 in food processing, agricultural technology, biotechnology and chemical process technology.

12. An application of a method as claimed in claim 7 or 8 in pharmaceutical research, manufacture and control of pharmaceuticals.

13. An application of a method as claimed in claim 7 or 8 in textile technology, and in the detergent and tenside industry.

14. A layer structure for the determination of a substance characterized in that at least one micellar recognition system is provided in a layer structure, which system comprises two non-miscible phases, at least one surface-active substance and at least one recognition component, that the micellar recognition system is homogeneously distributed in at least one single layer, and that at least one support is provided which is in contact with the layer structure and supports the transducing step and the gaining of information.

15. A layer structure as claimed in claim 14, characterized in that receptors, antibodies and enzymes and their synthetic and gene-manipulated hybrids, and fragments of all of these components are provided as recognition components.

16. A layer structure as claimed in claim 14 or 15, characterized in that auxiliary layers are provided in the layer structure in addition to the at least one single layer with the at least one micellar recognition system, which auxiliary layers improve biocompatibility and useful life, increase mechanical stability and/or serve as diffusion barrier and buffering layers.

17. A layer structure as claimed in one of the claims 14 to 16, characterized in that the sample with the substance is placed on any of the sides of the layer structure, and a support on any other side of the layer structure, with the support being used as a holding device.

18. A layer structure as claimed in one of the claims 14 to 17, characterized in that the two non-miscible phases are made up of a lipophilic phase of low polarity and a hydrophilic phase of increased polarity.

## Revendications

1. Procédé pour la détermination d'une substance, caractérisé en ce que l'on forme au moins un système de reconnaissance micellaire constitué de deux phases non miscibles, d'au moins une substance tensioactive, et d'au moins un composé de reconnaissance (1), que le système de reconnaissance micellaire est incorporé à une structure stratifiée (2) dans laquelle il constitue au moins une couche individuelle, que la structure stratifiée est mise en contact avec la substance (3), que dans cette couche individuelle il se produit au moins une étape de reconnaissance (4), que l'étape de reconnaissance est suivie par au moins une étape de transduction (5), que l'on obtient ainsi au moins une mesure (6) et que par cette mesure permet d'obtenir au moins une information sur la substance.

2. Procédé selon la revendication 1, caractérisé en ce que les propriétés physiques et chimiques de la couche individuelle sont ajustées à l'aide d'additifs modificateurs de la viscosité et de plastifiants.

3. Procédé selon la revendication 1 ou 2, caractérisé en ce que le composé de reconnaissance réagit avec la substance à déterminer en reconnaissant sélectivement la substance.

4. Procédé selon l'une des revendications de 1 à 3, caractérisé en ce que la structure stratifiée est constituée en outre de couches auxiliaires.

5. Procédé selon l'une des revendications de 1 à 4, caractérisé en ce que la substance est un ion, une molécule chargée, non chargée ou neutre, un isotope, un micro-organisme, un virus, une organelle, un sel ou un récepteur.

6. Procédé selon l'une des revendications de 1 à 5, caractérisé en ce que l'information concernant la substance est obtenue en tant que quantité, concentration, donnée numérique, ou activité.

7. Procédé selon l'une des revendications de 1 à 6, caractérisé en ce que le composé de reconnaissance réagit avec la substance à déterminer et que ceci est suivi par une étape de transduction.

8. Procédé selon la revendication 7, caractérisé en ce que le produit de la réaction entre le composé de reconnaissance et la substance à déterminer, donne lieu à une réaction sélective additionnelle mais limitée avec au moins un autre composant de reconnaissance durant la transduction.

9. Application d'un procédé selon la revendication 7 ou 8 dans les diagnostics médicaux et en analyse chimique clinique.

10. Application d'un procédé selon la revendication 7 ou 8 dans les techniques environnementales, les techniques de construction, les techniques spatiales, les techniques en salle blanche et dans les contrôles des eaux usées et des eaux potables.

11. Application d'un procédé selon la revendication 7 ou 8 dans la technologie alimentaire, dans la technologie agricole, dans la biotechnologie et dans les techniques de traitements chimiques.

12. Application d'un procédé selon la revendication 7 ou 8 dans la recherche pharmaceutique, dans la production de médicaments et dans le contrôle des médicaments.

13. Application d'un procédé selon la revendication 7 ou 8 dans les techniques de l'industrie textile et dans l'industrie des détergents et des tensioactifs.

14. Structure stratifiée pour la détermination d'une substance, caractérisée en ce que dans la structure stratifiée, il y au moins un système de reconnaissance micellaire, composé de deux phases non miscibles, d'au moins une substance tensioactive et d'au moins un composé de reconnaissance, que le système de reconnaissance micellaire est réparti de manière homogène dans au moins une couche individuelle et qu'au moins un vecteur est prévu, qui est lié à la structure stratifiée pour favoriser l'étape de transduction et l'obtention de l'information.

15. Structure stratifiée selon la revendication 14, caractérisée en ce que le composé de reconnaissance est un récepteur, un anticorps ou une enzymes, ou encore leur équivalent obtenu par synthèse ou par génie génétique, ou encore un fragments de ce composé.

16. Structure stratifiée selon la revendication 14 ou 15, caractérisée en ce que dans la structure stratifiée, à côté de la couche individuelle avec le système de reconnaissance micellaire, il y a des couches auxiliaires qui améliorent la biocompatibilité et la durée de vie, augmentent la solidité mécanique et/ou servent de barrière de diffusion et de couche tampon.

17. Structure de la couché selon l'une des revendications 14 à 16, caractérisée en ce que l'échantillon avec la substance est disposé sur un côté quelconque de la structure stratifiée et qu'un vecteur est disposé sur un autre côté quelconque, ce dernier servant de fixateur.

18. Structure de la couche selon l'une des revendications 14 à 17, caractérisée en ce que les deux phases non miscibles sont composées d'une phase lipophile de faible polarité et d'une phase hydrophile de polarité plus élevée.
